# EUROPÄISCHE PATENTSCHRIFT

(11) **EP 0 366 888 B1**
(45) Veröffentlichungstag und Bekanntmachung des Hinweises auf die Patenterteilung: **03.03.1993**
(21) Anmeldenummer: 89115167.2
(22) Anmeldetag: 17.08.1989
(51) Int. Cl.: A61K 33/14, A61K 9/06

(54) **Pharmazeutische Zubereitung zur Behandlung entzündeter Nasenschleimhäute**
Pharmaceutical preparation for treating inflammations of the nasal mucous membranes
Préparation pharmaceutique pour le traitement des inflammations des membranes muqueuses nasales

(30) Priorität: 23.09.1988 DE 3832401
(43) Veröffentlichungstag der Anmeldung: 09.05.1990
(73) Patentinhaber: CASSELLA Aktiengesellschaft, D-60386 Frankfurt (DE)
(72) Erfinder: Greve, Rainer, Dr., D-2360 Bad Segeberg (DE); Greve, Harald, Dr., D-5064 Rösrath 3 (DE); Pfadt, Joachim, D-2361 Todesfelde (DE)
(74) Vertreter: Urbach, Hans-Georg

(56) Entgegenhaltungen:
- EP-A- 0 053 754
- EP-A- 0 138 262
- FR-A- 2 520 234
- "ROTE LISTE 1967", Editio Cantor, Aulendorf/Württ, DE; Seite 614, "Inzellon"
- "ROTE LISTE 1982", Editio Cantor, Aulendorf/Württ, DE; Inzolen-HK/-KM21, Nr. 51093

## Beschreibung

Die vorliegende Erfindung betrifft eine pharmazeutische Zubereitung, die D-(+)-Pantothenylalko- hol (Dexpanthenol) und Kochsalz in physiologischer Konzentration enthält, sowie deren Verwendung zur Behandlung entzündeter Nasenschleimhäute.

Entzündete Nasenschleimhäute treten vor allem in der kalten Jahreszeit bei weiten Bevölkerungskreisen auf. Es ist bereits bekannt, daß physiologische Kochsalzlösung die nasale Schleimhautschwellung vermindert und so eine subjektive und objektive Besserung des Zustands der Patienten bewirkt (Spector et al, Clinical Allergy 12, 187 (1982)) und auch bei Säuglingen vorteilhaft verwendet werden kann (Albegger, Huber, Pädiatrie und Pädologie 20, 321 (1985)).

Ebenso ist der antientzündliche Effekt und die Erhöhung der Infektabwehr von Dexpanthenol bekannt (beispielsweise Weber, Deutsche Apotheker Zeitung 123, 1921 (1983)). Dexapanthenol ist auch bereits in Form einer Nasensalbe im Handel (Rote Liste 1988).

Die EP 53 754 sowie die FR-A 2 520 234 beschreiben jeweils ein Mittel zur Bekämpfung des Schnarchens, das in physiologischer Kochsalzlösung eine oberflächenaktive Substanz sowie gegebenenfalls auch Dexpanthenol enthält. Für die dort gewünschte Wirkung kommt insbesondere der oberflächenaktiven Substanz eine entscheidende Bedeutung zu. Zur Behandlung entzündeter Nasenschleimhäute ist dieses Mittel aber nicht geeignet, ja verbietet sich sogar, da oberflächenaktive Substanzen eine Wundheilung nicht fördern, sondern hinauszögern.

Es wurde nun überraschenderweise gefunden, daß eine pharmazeutische Zubereitung, die D-(+)-Pantothenylalkohol (Dexpanthenol) und Kochsalz in physiologischer Konzentration enthält, zur Behandlung entzündeter Nasenschleimhäute sehr viel besser geeignet ist als die bekannten Monopräparate und sich durch einen synergistischen Effekt auszeichnet.

Die erfindungsgemäßen Zubereitungen enthalten Dexpanthenol vorteilhafterweise in Mengen von 0,2 bis 20 Gew.%, vorzugsweise 5 bis 10 Gew.%.

Das Kochsalz liegt stets in physiologischer Konzentration vor, das heißt in einer Menge von 0,9 Gew.%.

Die pharmazeutischen Zubereitungen können flüssig oder dickflüssig bis halbfest sein. Sie können z.B. als Salben, Cremes oder Gele zum Einbringen in die Nase oder als Lösungen zum Tropfen oder Sprühen vorliegen.

Als Träger für flüssige Darreichungsformen eignen sich insbesondere wäßrige Systeme mit oder ohne Zusatz von Glycerol, Sorbitol und anderen Polyolen.

Als Trägerstoffe für dickflüssige oder halbfeste pharmazeutische Zubereitungen, wie z.B. Salben, Cremes oder Gelen, eignen sich z.B. Paraffinkohlenwasserstoffe, Vaseline, Wollwachsprodukte und andere pharmazeutisch verwendbare viskositätserhöhende Grundstoffe, bei hydrophilen Gelen z.B. Wasser, Glycerol oder Sorbitol, die mit geeigneten Quellstoffen, wie z.B. Polyacrylsäure, Zellulosederivaten, Stärke oder Traganth geliert werden.

Die pharmazeutischen Zubereitungen können neben den Wirk- und Trägersubstanzen und gegebenenfalls vorhandenen Emulgatoren noch andere pharmazeutisch unbedenkliche und mit den Wirkstoffen verträgliche Hilfs- und/oder Zusatzstoffe, wie z.B. Füll-, Streck-, Binde-, Netz-, Stabilisierungs-, Färbe-, Puffer- und Riechstoffe enthalten. Weiterhin können mikrobiologisch aktive chemische Verbindungen, wie z.B. Konservierungsstoffe oder Antiseptica, zur Verbesserung der mikrobiellen Stabilität in den pharmazeutisch üblichen Konzentrationen in den Zubereitungen enthalten sein.

Darüber hinaus können die erfindungsgemäßen Zubereitungen auch noch eine oder mehrere andere pharmakologisch wirksame Substanzen enthalten. Insbesondere können noch weitere Verbindungen mit Pantothensäure-Wirkung in freier Form und/oder in Form von Derivaten enthalten sein.

Die Herstellung der erfindungsgemäßen Zubereitungen erfolgt durch Mischen bzw. Lösen der Wirksubstanzen in der pharmakologisch wirksamen Konzentration, der Hilfs- und/oder Zusatzstoffe sowie der gegebenenfalls weiteren pharmakologisch wirksamen Substanzen in dem vorgesehenen Trägermedium.

Zum Nachweis der synergistischen Wirkungsqualität der erfindungsgemäßen Zubereitungen wurde in Versuchen jeweils eine Nasenseite mit physiologischer Kochsalzlösung allein und die andere Nasenseite mit einer erfindungsgemäßen Zubereitung in Form einer wäßrigen Lösung behandelt.

Diese Versuche wurden in einer anderen Versuchsreihe wiederholt, wobei die physiologische Kochsalzlösung durch ein Dexpanthenol-Monopräparat ersetzt wurde.

In beiden Versuchsreihen ergab die laufende Rhinoskopie bei den mit der erfindungsgemäßen Zubereitung behandelten Nasenseiten eine deutliche Verringerung von Läsionen der Nasenschleimhäute und Nasenflügel-Innenflächen sowie eine signifikante Reduktion von Mikroblutungen und Borkenbildung.

Bei weiteren Versuchen, bei denen beide Nasenseiten mit der erfindungsgemäßen Zubereitung behandelt wurden, konnte eine deutliche Herabsetzung der Akut-Symptomatik, wie z.B. Schmerzen oder Nasenbluten, erreicht werden. Die Summe aller unangenehmen Symptome wurde insgesamt als weniger lästig empfungen als bei der Behandlung mit jeweils nur einem Monopräparat.

Bei der Verwendung der erfindungsgemäßen Zubereitungen sind aufgrund des bisher vorliegenden wissenschaftlichen Erkenntnismaterials über die einzelnen Wirkstoffe keine gravierenden Nebenwirkungen zu erwarten. In den bisher durchgeführten Versuchen konnten ebenfalls keine Nebenwirkungen beobachtet werden.

Die folgenden Ausführungsbeispiele veranschaulichen die Herstellung flüssiger und halbfester erfindungsgemäßer Zubereitungen.

### Beispiel 1:

Zur Herstellung von 100 g einer klaren wäßrigen Lösung werden in einem Glasgefäß mit Rühreinrichtung 0,9 g Natriumchlorid und 5 g Dexpanthenol mit 90 g gereinigtem Wasser übergossen und die Substanzen durch gründliches Rühren klar gelöst. Der Lösung werden 0,02 g Benzalkoniumchlorid als Konservierungsstoff zugesetzt und gleichfalls unter Rühren gelöst. Zuletzt wird mit weiterem Wasser zum Endgewicht von 100,0 g aufgefüllt und homogen gerührt. Gegebenenfalls muß die Lösung über neutrale Zellulosefilter filtriert werden. Die klare, geruchlose Flüssigkeit wird in Enghalsflaschen aus Braunglas zu 10 oder 20 ml abgefüllt, welche wahlweise mit einer Tropfpipette oder einer Sprüh-Dosierpumpe ausgerüstet werden können. Anwendung: Mehrmals täglich nach Bedarf einen Tropfen bzw. einen Sprühstoß in jedes Nasenloch geben und aufziehen.

### Beispiel 2:

Zur Herstellung von 100 g einer klaren gepufferten wäßrigen Lösung mit verstärkter Pantothensäure-Wirkung werden in einem Glasgefäß mit Rühreinrichtung 0,9 g Kochsalz und 7,5 g Dexpanthenol mit 85 g gereinigtem Wasser übergossen und durch gründliches Rühren klar gelöst. Zur Einstellung eines stabilen pH-Wertes von 5,3 werden der Lösung 0,756 g Kaliumdihydrogenphosphat und 0,024 g Natriummonohydrogenphosphat-Dodecahydrat als Puffersubstanzen sowie zur Konservierung 0,02 g Benzalkoniumchlorid zugesetzt und unter Rühren gelöst. Die Lösung wird mit weiterem Wasser zum Endgewicht von 100,0 g aufgefüllt und homogen gerührt. Gegebenenfalls wird über neutrale Zellulosefaser filtriert. Die klare, geruchlose Flüssigkeit wird in Enghalsflaschen aus Braunglas zu 10 oder 20 ml abgefüllt, welche wahlweise mit einer Tropfpipette oder einer Sprüh-Dosierpumpe ausgerüstet werden können.

Anwendung: 3 bis 5 mal täglich oder nach Bedarf einen Tropfen oder einen Sprühstoß in jedes Nasenloch geben und aufziehen.

### Beispiel 3:

Zur Herstellung von 100 g einer klaren gepufferten wäßrigen Lösung mit erhöhter Haftfähigkeit werden 0,9 g Kochsalz und 5 g Dexpanthenol sowie 0,02 g Benzalkoniumchlorid als Konservierungsstoff in einem Glasgefäß mit Rühreinrichtung mit 80 g gereinigtem Wasser übergossen und durch Rühren klar gelöst. Zur Einstellung eines stabilen pH-Wertes von 5,3 werden der Lösung 0,756 g Kaliumdihydrogenphosphat und 0,024 g Natriummonohydrogenphosphat-Dodecahydrat zugesetzt und unter Rühren gelöst. Zur Herabsetzung der Oberflächenspannung und Erhöhung der Viskosität werden der Lösung noch 5 g Sorbitol-Lösung 70% und 5 g Glycerol 85% zugesetzt und die Mischung mit weiterem Wasser zum Endgewicht von 100,0 g aufgefüllt. Zuletzt wird bis zur Schlierenfreiheit homogen gerührt und gegebenenfalls über neutrale Zellulosefilter filtriert. Die klare, geruchlose Flüssigkeit wird in Enghalsflaschen aus Braunglas zu 10 oder 20 ml abgefüllt, welche wahlweise mit einer Tropfpipette oder einer Sprüh-Dosierpumpe ausgerüstet werden können. Anwendung: Mehrmals täglich nach Bedarf einen Tropfen bzw. einen Sprühstoß in jedes Nasenloch geben und aufziehen.

### Beispiel 4:

Zur Herstellung von 100 g einer Lösung nach Beispiel 3, jedoch mit verstärkter Pantothensäure-Wirkung, werden in einem Glasgefäß mit Rühreinrichtung 0,9 g Kochsalz und 10 g Dexpanthenol sowie 0,02 g Benzalkoniumchlorid als Konservierungsstoff mit 75 g gereinigtem Wasser übergossen und durch gründliches Rühren klar gelöst. Zur Herabsetzung der Oberflächenspannung und Erhöhung der Viskosität und Haftfähigkeit werden der Lösung noch 5 g Sorbitol-Lösung und 5 g Glycerol 85% zugesetzt und die Mischung mit weiterem Wasser zum Endgewicht von 100,0 g aufgefüllt. Dann wird bis zur Schlierenfreiheit homogen gerührt und gegebenenfalls über neutrale Zellulosefilter filtriert. Die klare, geruchlose Flüssigkeit wird in Enghalsflaschen aus Braunglas zu 10 oder 20 ml abgefüllt, welche wahlweise mit einer Tropfpipette oder einer Sprüh-Dosierpumpe ausgerüstet werden können. Anwendung: 3 bis 5 mal täglich oder nach Bedarf einen Tropfen bzw. einen Sprühstoß in jedes Nasenloch geben und aufziehen.

### Beispiel 5:

Zur Herstellung von 100 g eines dickflüssigen Gels läßt man in einem verschließbaren Glasgefäß 0,625 g Polyacrylsäure in 50 g gereinigtem Wasser zu einem Sol quellen. Nach etwa 24 Stunden werden unter intensivem Rühren 0,1 g Ammoniaklösung 10% eingetragen, wobei Gelbildung einsetzt. In einem zweiten Glasgefäß werden sodann 0,9 g Kochsalz und 5 g Dexpanthenol sowie 0,02 g Benzalkoniumchlorid als Konservierungsstoff in 40 g gereinigtem Wasser gelöst und die klare Lösung wiederum unter intensivem Rühren langsam in das Polyacrylatgel eingetragen. Zuletzt wird mit weiterem Wasser zum Endgewicht von 100,0 g aufgefüllt und das Produkt bis zur Homogenität gerührt. Man erhält ein fast klares, farb- und geruchloses dickflüssiges Gel, welches in kleine Tuben zu 5 oder 10 g, mit ausgezogener Applikationsspitze, abgefüllt wird. Anwendung: Nach Bedarf mehrmals täglich eine kleine Portion Gel möglichst hoch in beide Nasenlöcher einbringen und aufziehen.

### Beispiel 6:

Zur Herstellung einer cremigen Nasensalbe werden in einem Glasgefäß mit Rührstab 0.9 g Kochsalz und 5 g Dexpanthenol sowie zur Konservierung 0,02 g Benzalkoniumchlorid in gereinigtem Wasser zu 30,0 g gelöst. In einer SalbenReibschale mit Pistill wird die wäßrige Wirkstoff-Lösung in 70 g Wollwachsalkohol-Salbe eingearbeitet und unter kräftigem Rühren fein emulgiert. Man erhält eine weiße, weiche Nasensalbe, welche in kleine Tuben zu 5 oder 10 g, mit ausgezogener Applikationsspitze, abgefüllt wird. Anwendung: Nach Bedarf mehrmals täglich eine kleine Portion Salbe in jedes Nasenloch geben und nach Erweichung durch die Körpertemperatur aufziehen.

## Patentansprüche (Patentansprüche für folgende(n) Vertragsstaat(en) : AT, BE, CH, DE, FR, GB, IT, NL)

1. Pharmazeutische Zubereitung zur Behandlung von entzündeten Wasenschleimhäuten, dadurch gekennzeichnet, daß sie D-(+)-Pantothe- nylalkohol und Kochsalz in physiologischer Konzentration als alleinige Wirksubstanzen enthält.

2. Pharmazeutische Zubereitung gemäß Anspruch 1, dadurch gekennzeichnet, daß sie D-(+)-Pantothenylalkohol in Mengen von 0,2 bis 20 Gew.%, vorzugsweise 5 bis 10 Gew.%, enthält.

3. Pharmazeutische Zubereitung gemäß Anspruch 1 und/oder 2, dadurch gekennzeichnet, daß sie flüssig oder dickflüssig bis halbfest ist.

4. Pharmazeutische Zubereitung gemäß einem oder mehreren der Ansprüche 1 bis 3, dadurch gekennzeichnet, daß sie neben den Wirk- und Trägerstoffen und gegebenenfalls Emulgatoren noch andere pharmazeutisch unbedenkliche und mit den Wirkstoffen verträgliche Hilfs-und/oder Zusatzstoffe enthält.

5. Verfahren zur Herstellung der pharmazeutischen Zubereitungen der Ansprüche 1 bis 4, dadurch gekennzeichnet, daß die Wirksubstanzen in der pharmakologisch wirksamen Konzentration zusammen mit den Hilfs- und/oder Zusatzstoffen in dem vorgesehenen Trägermedium gemischt bzw. gelöst werden.

## Patentansprüche (Patentansprüche für folgende(n) Vertragsstaat(en) : ES)

1. Verfahren zur Herstellung einer pharmazeutischen Zubereitung zur Behandlung von entzündeten Wasenschleimhäuten, die D-(+)-Pantothenylalkohol und Kochsalz in physiologischer Konzentration als alleinige Wirksubstanzen enthält, dadurch gekennzeichnet, daß die Wirksubstanzen in der pharmakologisch wirksamen Konzentration in dem vorgesehenen Trägermedium gemischt bzw. gelöst werden.

2. Verfahren gemäß Anspruch 1, dadurch gekennzeichnet, daß D-(+)-Pantothenylalkohol in Mengen von 0,2 bis 20 Gew.%, vorzugsweise 5 bis 10 Gew.%, beigemischt bzw. gelöst wird.

3. Verfahren gemäß Anspruch 1 und/oder 2, dadurch gekennzeichnet, daß die Zubereitung flüssig oder dickflüssig bis halbfest ist.

4. Verfahren gemäß einem oder mehreren der Ansprüche 1 bis 3, dadurch gekennzeichnet, daß die Zubereitung neben den Wirk- und Trägerstoffen und gegebenenfalls Emulgatoren noch andere pharmazeutisch unbedenkliche und mit den Wirkstoffen verträgliche Hilfs-und/oder Zusatzstoffe enthält.

## Claims (Claims for the following Contracting State(s) : AT, BE, CH, DE, FR, GB, IT, LI, NL)

1. Pharmaceutical preparation for treating inflamed nasal mucous membranes, characterized in that it contains as sole active substances D-(+)-pantothenyl alcohol and sodium chloride in physiological concentration.

2. Pharmaceutical preparation according to claim 1, characterized in that it contains D-(+)-pan- tothenyl alcohol in amounts of 0.2 to 20 % by weight, preferably 5 to 10 % by weight.

3. Pharmaceutical preparation according to claim 1 and/or 2, characterized in that it is liquid or viscous to semi-solid.

4. Pharmaceutical preparation according to one or more of claims 1 to 3, characterized in that it contains, in addition to the active substances and carriers and optionally emulsifiers, other auxiliaries and/or additives that are pharmaceutically acceptable and compatible with the active substances.

5. Process for preparing the pharmaceutical preparations of claims 1 to 4, characterized in that the active substances are mixed or dissolved in the chosen carrier medium in their pharmacologically effective concentration together with the auxiliaries and/or additives.

## Claims (Claims for the following Contracting State(s) : ES)

1. Process for preparing a pharmaceutical preparation for treating inflamed nasal mucous membranes which contains as sole active substances D-(+)-pantothenyl alcohol and sodium chloride in physiological concentration, characterized in that the active substances are mixed or dissolved in the chosen carrier medium in their pharmacologically effective concentration.

2. Process according to claim 1, characterized in that D-(+)-pantothenyl alcohol is admixed or dissolved in amounts of 0.2 to 20 % by weight, preferably 5 to 10 % by weight.

3. Process according to claim 1 and/or 2, characterized in that the preparation is liquid or viscous to semi-solid.

4. Process according to one or more of claims 1 to 3, characterized in that the preparation contains, in addition to the active substances and carriers and optionally emulsifiers, other auxiliaries and/or additives that are pharmaceutically acceptable and compatible with the active substances.

## Revendications (Revendications pour l'(les) Etat(s) contractant(s) suivant(s) : suivants : AT, BE, CH, DE, FR, GB, IT, LI, NL)

1. Préparation pharmaceutique pour le traitement de muqueuses nasales enflammées, caractérisée en ce qu'elle comprend comme seules substances actives de l'alcool D-(+)-pantothé- nylique avec du chlorure de sodium à la teneur physiologique.

2. Préparation selon la revendication 1 caractérisée en ce qu'elle contient l'alcool D-(+)-panto- thénylique dans des proportions de 0,2 à 20 % en poids, de préférence de 5 à 10 %.

3. Préparation selon la revendication 1 et/ou 2, caractérisée en ce que c'est un liquide fluide ou épais ou un produit semi-solide.

4. Préparation selon une ou plusieurs des revendications 1 à 3, caractérisée en ce qu'elle comprend encore, en plus des matières actives et des véhicules et le cas échéant d'émul- sionnants, d'autres adjuvants et/ou additifs sans inconvénient du point de vue pharmaceutique et compatibles avec les matières actives.

5. Procédé de préparation d'une préparation selon les revendications 1 à 4, procédé caractérisé en ce que l'on mélange ou dissout dans le milieu choisi les matières actives à la concentration pharmacologiquement efficace, avec les adjuvants et/ou additifs.

## Revendications (Revendications pour l'(les) Etat(s) contractant(s) suivant(s) : suivant : ES)

1. Procédé d'obtention d'une préparation pharmaceutique destinée à traiter des muqueuses nasales enflammées, comprenant comme seules matières actives de l'alcool D-(+)-pantothényli- que et du chlorure de sodium à la concentration physiologique, procédé caractérisé en ce que l'on mélange ou dissout dans le milieu véhicule choisi les matières actives à la concentration pharmacologiquement efficace.

2. Procédé selon la revendication 1, caractérisé en ce que l'on mélange ou dissout l'alcool D-(+)-pantothénylique dans des proportions de 0,2 à 20 % en poids, de préférence de 5 à 10 %.

3. Procédé selon la revendication 1 et/ou 2, caractérisé en ce que la préparation est une préparation liquide fluide ou épaisse à semi-solide.

4. Procédé selon une ou plusieurs des revendications 1 à 3, caractérisé en ce que la préparation contient en outre, à côté des matières actives et véhicules et le cas échéant d'émul- sionnants, d'autres adjuvants et/ou additifs sans inconvénient sur le plan pharmaceutique et compatibles avec les matières actives.
